# EUROPEAN PATENT APPLICATION

(11) **EP 3 970 717 A1**
(43) Date of publication of application: **23.03.2022**
(21) Application number: 21190311.7
(22) Date of filing: 24.06.2019
(51) Int. Cl.: A61K 31/402, A61P 13/12

(54) **TREATMENT OF PROTEINURIA**

(30) Priority: 22.06.2018 EP 18179319
(62) Divisional of application: 19734023.5
(71) Applicant: SynAct Pharma ApS, 2840 Holte (DK)
(72) Inventor: Jonassen, Thomas Engelbrecht Nordkild, 2840 Holte (DK)
(74) Representative: Høiberg P/S

(57) **Abstract**

Provided is a composition comprising a phenyl pyrrole aminoguanidine derivative for use in a method of treating kidney disease, such as nephrotic syndromes.

## Description

### Technical field

The present invention relates to a composition comprising a compound of formula (I), (Ia) or (II), or a pharmaceutically acceptable derivative thereof, for use in a method of treating kidney disease, especially proteinuric kidney diseases, such as nephrotic syndromes.

### Background

Proteinuria, the hallmark of glomerular injury, is a common finding on urinalysis, and is by itself a strong, independent and modifiable risk factor for end stage renal disease, premature death of cardiovascular origin, and ischemic stroke in patients with diabetes. Despite recent advances refractory proteinuria continues to be a challenge in clinical practice. It is imperative to develop more effective modalities to ameliorate glomerular injury and induce remission of proteinuria. Recent evidence points to melanocortin system as a novel target for treatment of proteinuria.

Membranous nephropathy (MN) is one of the most common causes of nephrotic syndrome in adults. One-third of the patients have a good prognosis with spontaneous remission. Even so, approximately 50% of the remainder manifest an unchanged disease state, whereas 50% progress into ESRD (end-stage renal disease) and dialysis. The nephrotic syndrome is a glomerular disease characterized by proteinuria, edema, hypoalbuminemia, and hyperlipidemia. Most MN cases are idiopathic and the mechanisms underlying the disease are still largely unknown. Treatments have classically been nonspecific and often include steroids with anti-inflammatory actions, sometimes in combination with cytotoxic agents. These drugs affect multiple tissues, causing cytotoxic effects, osteoporosis, adrenal insufficiency, hypertension, peptic ulcers, and increased risk of glucose intolerance and infections. Therefore, more effective and specific treatment options are needed.

The melanocortin system is a set of neuropeptidergic and immunoendocrine signaling pathways that play an integral role in the homeostatic control of a diverse array of physiological functions, including melanogenesis, stress response, inflammation, immunomodulation and adrenocortical steroidogenesis. It consists of multiple components, including the five G protein-coupled melanocortin receptors: melanocortin receptor 1 (MC1R) to MC5R; peptide ligands: α, β, γ-melanocyte stimulating hormone (α, β, γ- MSH), adrenocorticotropic hormone (ACTH) secreted by the anterior pituitary; and endogenous antagonists. The biological functions of melanocortin system are mediated by the five melanocortin receptors (MCRs), which have distinct tissue distribution, convey different signalling and exert varying biological activities in different organ systems.

Adrenocorticotropic hormone (ACTH) is an endogenous peptide hormone and agonist for all melanocortin receptors 1 to 5 (MC1-5R), of which MC2R specifically binds ACTH; steroidogenesis is triggered only by ACTH and mediated via MC2R in the adrenal cortex. Alpha-melanocyte stimulating hormone (αMSH) is a small endogenous peptide hormone, structurally related to ACTH, which binds all of the MCRs except MC2R. MC1R, abundantly expressed by melanocytes in the skin, is a key control point in melanogenesis and determines hair colour.

Melanocortin therapy by using ACTH or non-steroidogenic melanocortin peptides attenuates proteinuria and glomerular injury in experimental glomerular diseases and induces remission of nephrotic syndrome in patients with diverse glomerulopathies, even those resistant to steroids, including membranous nephropathy (MN), minimal change disease (MCD), focal segmental glomerulosclerosis (FSGS) and IgA nephropathy. The underlying mechanism remains elusive, but the role of melanocortin 1 receptor (MC1R) has been implicated and MC1R is heavily and specifically expressed in human kidney cortex and specific renal cell types including podocytes (Lindskog et al. 2010). However, recent studies have found effects of ACTH monotherapy and NDP-aMSH ([NIe₄, D-Phe₇]-αMSH) also in patients bearing dominant-negative MC1R mutations, which was confirmed also in MC1R-null mice and in vitro in primary podocytes derived from MC1R-null and wildtype mice. The antiproteinuric effect of melanocortin signalling thus appears to be steroidogenicindependent and MC1R-dispensable, and the melanocortin effect might, at least in part, target a pathogenic pathway common to all proteinuric kidney diseases. Podocytes are a critical component of the glomerular filtration barrier controlling glomerular permeability and selectivity and are considered a major culprit accounting for massive proteinuria in diverse glomerular diseases. The beneficial effect of melanocortin therapy is likely attributable to a direct action on podocytes (Qiao et al. 2016).

It is at present therefore not possible to predict which melanocortin agonists and which associated targets will present as useful candidates for treatment of proteinuric diseases such as kidney diseases.

Phenyl pyrrole aminoguanidine derivatives with activity on the melanocortin receptors are disclosed in WO 2007/141343. One example of such compound is the anti-inflammatory AP1189 ((*E*)-*N*-*trans*-{3-[1-(2-nitrophenyl)-1H-pyrrol-2-yl]-allylidene}-aminoguanidium acetate) which was first shown to bind the MC1R (WO 2007/141343) and later was identified as a biased dual agonist at receptors MC1R and MC3R that does not provoke canonical cAMP generation (and hence no MC1R-induced melanogenesis) but instead appear to induce alternative pathways including ERK1/2-phosphorylation and Ca²⁺ mobilization (Montero-Melendez et al. 2015).

### Summary

The present inventors have found that the phenyl pyrrole aminoguanidine derivative AP1189 ((*E*)-*N*-*trans*-{3-[1-(2-nitrophenyl)-1H-pyrrol-2-yl]-allylidene}-aminoguanidium acetate) significantly reduces proteinuria in Passive Haymann Nephritis, a nephropathy model, as compared to vehicle, measured as a significant reduction of albumin excretion rate and FR_{Alb} (fraction albumin excretion); and also has a significantly higher GFR (glomerular filtration rate; creatinine clearance) and a significantly reduced FR_{Alb} compared to treatment with ACTH(1-24). This implies that AP1189 and related compounds are candidates for the improved treatment of proteinuric kidney diseases.

It is an aspect of the present disclosure to provide a composition comprising a compound of formula (I) or (Ia): 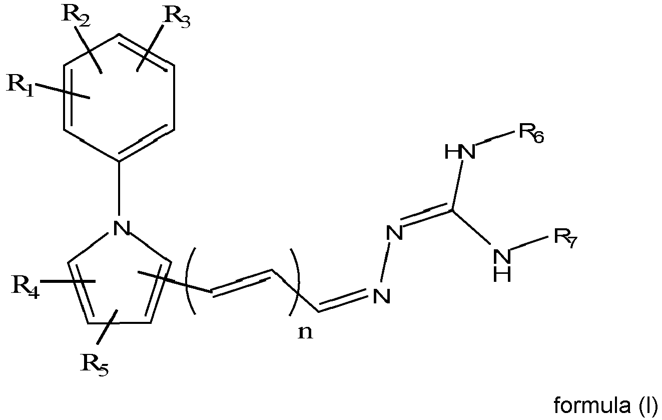 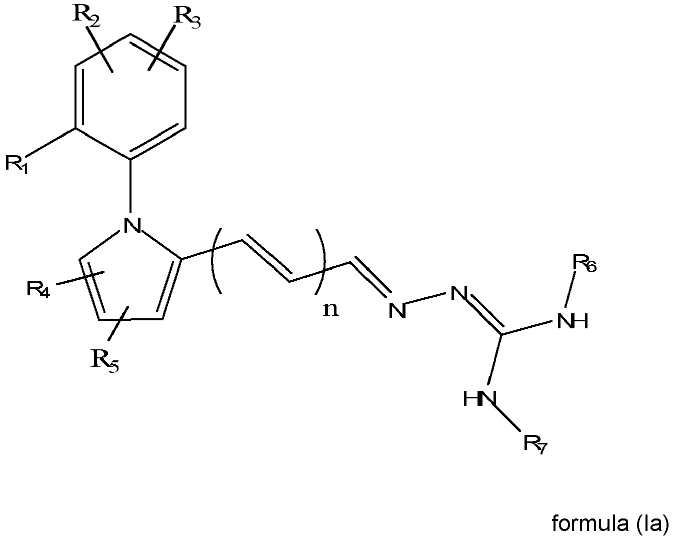 including tautomeric and isomeric forms, such as enantiomeric forms, diastereomeric forms and racemic forms, thereof;
wherein
n is 1, 2 or 3;
each R₁, R₂, R₃, R₄ and R₅ is independently selected from the group consisting of hydrogen, optionally substituted C₁₋₆-alkyl, optionally substituted C₃₋₆-cycloalkyl, optionally substituted C₂₋₆-alkenyl, optionally substituted C₄₋₆-alkadienyl, optionally substituted C₂₋₆- alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆- alkylcarbonyl, formyl, C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, amino, monoand di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆- alkylamino-C₁₋₆-alkyl-aminocarbonyl, C₁₋₆-alkylcarbonylamino, amino-C₁₋₆-alkyl- carbonylamino, mono- and di(C₁₋₆-alkyl)amino-C₁₋₆-alkyl-carbonylamino, cyano, guanidino, carbamido, C₁₋₆-alkanoyloxy, C₁₋₆-alkylsulphonyl, C₁₋₆-alkylsulphinyl, C₁₋₆-alkylsulphonyl- oxy, aminosulfonyl, mono- and di(C₁₋₆-alkyl)aminosulfonyl, nitro, optionally substituted C₁₋₆-alkylthio and halogen, where any nitrogen-bound C₁₋₆-alkyl is optionally substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine;
each R₆ and R₇ is independently selected from the group consisting of hydrogen, optionally substituted C₁₋₆-alkyl, optionally substituted C₂₋₆-alkenyl, optionally substituted C₄₋₆- alkadienyl, optionally substituted C₂₋₆-alkynyl, optionally substituted C₁₋₆-alkoxycarbonyl, optionally substituted C₁₋₆-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di(C₁₋₆-alkyl)aminocarbonyl, amino- C₁₋₆-alkyl-aminocarbonyl and mono- and di(C₁₋₆-alkyl)amino- C₁₋₆-alkyl-aminocarbonyl; or R₆ and R₇ may together form a five- or six-membered nitrogen-containing ring;
or a pharmaceutically acceptable derivative thereof, for use in the treatment of a kidney disease.

It is also an aspect of the present disclosure to provide a composition comprising a compound of formula (II): 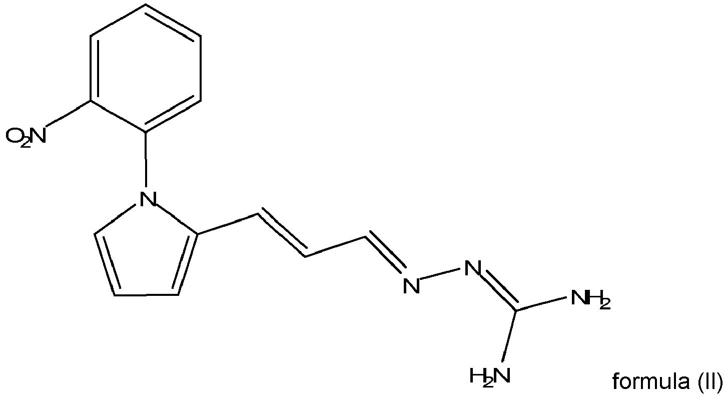
including tautomeric and isomeric forms, such as enantiomeric forms, diastereomeric forms and racemic forms, thereof;
or a pharmaceutically acceptable derivative thereof, for use in the treatment of a kidney disease.

It is also an aspect of the present disclosure to provide a composition comprising a compound selected from the group consisting of {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; (*E*)-*N*-*trans*-{3-[1-(2-nitrophenyl)-1H-pyrrol-2-yl]-allylidene}-aminoguanidine; and (*E*)-*N*-*trans*-{3-[1-(2-nitrophenyl)-1H-pyrrol-2-yl]-allylidene}-aminoguanidinium acetate, or a pharmaceutically acceptable derivative thereof, for use in the treatment of a kidney disease.

In one embodiment said kidney disease is a kidney disease that present with proteinuria.

In one embodiment said kidney disease is a glomerular disease, such as a disease affecting the podocytes of the glomeruli.

In one embodiment said kidney disease is nephrotic syndrome (glomerulonephrosis), such as primary nephrotic syndrome or secondary nephrotic syndrome.

### Definitions

The term "pharmaceutically acceptable derivative" in the present context includes pharmaceutically acceptable salts, which indicate a salt which is not harmful to the patient. Such salts include pharmaceutically acceptable basic or acid addition salts as well as pharmaceutically acceptable metal salts, ammonium salts and alkylated ammonium salts. A pharmaceutically acceptable derivative further includes esters and prodrugs, or other precursors of a compound which may be biologically metabolized into the active compound, or crystal forms of a compound.

The term "acid addition salt" is intended to include "pharmaceutically acceptable acid addition salt" which indicates salts which are not harmful to the patient. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 66, 2, (1977) which is incorporated herein by reference.

The term "therapeutically effective amount" of a compound as used herein refers to an amount sufficient to cure, alleviate, prevent, reduce the risk of, or partially arrest the clinical manifestations of a given disease or disorder and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

The terms "treatment" and "treating" as used herein refer to the management and care of a patient for the purpose of combating a condition, disease or disorder. The term is intended to include the full spectrum of treatments for a given condition from which the patient is suffering. The patient to be treated is preferably a mammal, in particular a human being. Treatment of animals, such as mice, rats, dogs, cats, horses, cows, sheep and pigs, is, however, also within the scope of the present context. The patients to be treated can be of various ages.

### Description of Drawings

Figure 1: Rats treated with test compound 1 (AP1189; (*(E)-N-trans*-{3-[1-(2-nitrophenyl)-1H-pyrrol-2-yl]-allylidene}-aminoguanidium acetate) have a significant reduction in proteinuria at day 28 and day 42 compared to control rats (vehicle).
Control rats maintained an unchanged albumin excretion at all time points, whereas rats treated with test compound 1 show a 31±8% reduction in albumin excretion rate at day 28 and a 48±10% reduction in albumin excretion rate at day 482. See Examples for details.
Figure 2A) Rats treated with test compound 1 (AP1189; (*(E)-N-trans-*{3-[1-(2-nitrophenyl)-1H-pyrrol-2-yl]-allylidene}-aminoguanidium acetate) have a significantly higher GFR (creatinine clearance) following 4 weeks treatment than the rats treated with the positive control (ACTH 1-24):
   i) positive control at 10 mg/day: 2.19 ±0.24 mL/min
   ii) test compound 1 at 20 mg/kg: 3.12±0.14 mL/min (p=0.002)
   iii) test compound 1 at 40 mg/kg: 2.94±0.14 mL/24 min (p=0.01).
Figure 2B) Rats treated with test compound 1 (AP1189; (*(E)-N-trans-*{3-[1-(2-nitrophenyl)-1H-pyrrol-2-yl]-allylidene}-aminoguanidium acetate) have a significantly reduced fraction albumin excretion (FEAlb) compared to positive control (ACTH 1-24):
   i) positive control at 10 mg/day: 0.59±0.02%
   ii) test compound 1 at 20 mg/kg: 0.045±0.04% (p=0.007)
   iii) test compound 1 at 40 mg/kg: 0.35±0.05% (p=0.001).

### Detailed description

Melanocortin (MC) receptors (MC1R-MC5R), a family of class A G protein-coupled receptors (GPCRs), are attractive therapeutic targets for a number of conditions due to their wide distribution and diversity of physiological processes they regulate. MC1R regulates UV light-induced skin tanning and other immune responses because of its expression on leukocytes. MC2R regulates cortisol production on the adrenal glands, whereas MC5R plays a role on exocrine glands secretions. MC3R and MC4R exert non-redundant functions on energy homeostasis in addition to specific anti-inflammatory roles; whereas MC3R activation is particularly protective for joint inflammation such as arthritis, MC4R provides neuroprotection in brain inflammation. Accordingly, an array of pathological situations could be targeted with MCR-drugs including skin conditions, cardiovascular pathologies, joint inflammation, obesity and cachexia.

Peripheral MC1R and MC3R can be pharmacologically activated to induce antiinflammation. The endogenous agonist α-melanocyte―stimulating hormone (aMSH), like other protective mediators, is released by immune cells to counterbalance proinflammatory signals, thus preventing excessive tissue damage. In line with the resolution of inflammation concept, therapeutics targeting MC1R and MC3R act by mimicking the body's own protective resources and might be characterized by a lighter burden of side effects.

Shown to be effective in rheumatic diseases since the early 1950s, the use of corticotropin or adrenocorticotropin hormone (ACTH) declined when synthetic glucocorticoids became available. However, the discovery of an alternative anti-inflammatory mechanism for ACTH involving activation of peripheral MC receptors on immune cells has revived the interest in developing novel ACTH-like molecules with no steroidogenic effects for the treatment of joint diseases such as gout or RA (rheumatoid arthritis). However, the limitation in the translational delivery of novel MC drugs besides the marketed ACTH formulations is imposed by the lack of receptor selectivity achieved so far.

Innovative approaches in G protein-coupled receptor drug discovery might help to overcome this limitation. Allosteric modulation consists in the ability of a molecule to enhance (positive modulation) or reduce (negative modulation) the effect of the endogenous ligand by binding to a distinct site of the receptor protein, termed allosteric site. A higher degree of selectivity is expected as allosteric regions are less conserved among the five MCRs, and indeed, allosteric modulators at MC4R are currently under development for the treatment of obesity.

Another emerging concept of significant therapeutic interest is the one of biased agonism. The obsolete notion that receptors could exist in two unique conformations, the active one and the inactive one, has been replaced with the conception that multiple active conformations can exist, each one creating a distinct signal yielding multiple functional outcomes. Receptor activation, rather than linear and static, is emerging as a highly dynamic and multidimensional process in which a diversity of active conformations may be induced by different molecules leading to distinct effects.

The small molecule AP1189 ((*E*)-*N*-*trans*-{3-[1-(2-nitrophenyl)-1H-pyrrol-2-yl]-allylidene}-aminoguanidium acetate) has been characterized as a biased agonist at receptors MC1R and MC3R, which does not induce canonical cAMP generation, but cause ERK1/2 phosphorylation, a signaling responsible for the proefferocytic effect evoked in mouse primary macrophages. AP1189 was shown to reduce cytokine release in macrophages, whereas no melanogenesis was induced by AP1189 in melanocytes. In vivo, oral AP1189 elicits anti-inflammatory actions in peritonitis and accelerated the resolution phase, and afforded significant reduction of macroscopic and histological parameters of joint disruption in experimental inflammatory arthritis. AP1189 is thus a biased dual agonist at MC1R and MC3R with anti-inflammatory properties together with a lack of effect on melanogenesis.

Melanocortin therapy by using adrenocorticotropic hormone (ACTH) or non-steroidogenic melanocortin peptides attenuates proteinuria and glomerular injury in experimental glomerular diseases and induces remission of nephrotic syndrome in patients with diverse glomerulopathies, even those resistant to steroids.

Proteinuria, the hallmark of glomerular injury, is a common finding on urinalysis, and is by itself a strong, independent and modifiable risk factor for end stage renal disease, premature death of cardiovascular origin, and ischemic stroke in patients with diabetes. Despite recent advances refractory proteinuria continues to be a challenge in clinical practice.

It is an aspect of the present disclosure to provide a composition comprising a compound of formula (I) or (Ia): 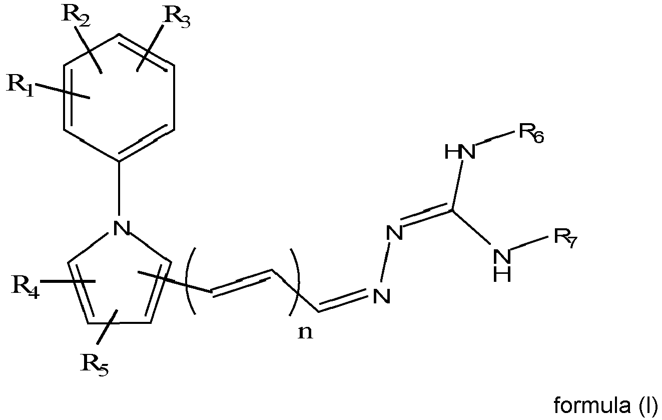 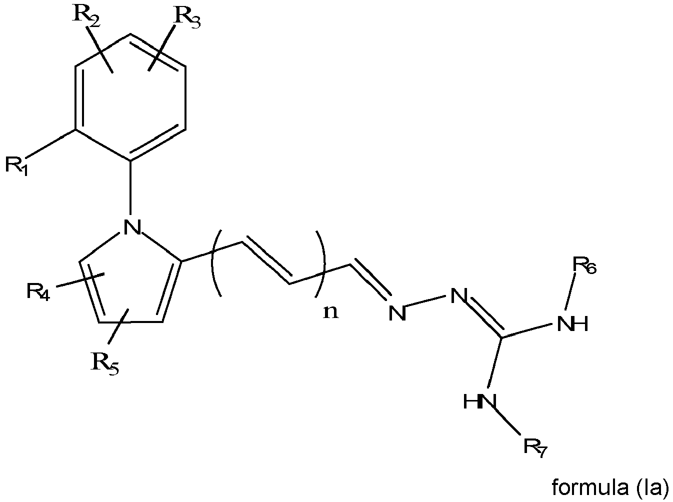
including tautomeric and isomeric forms, such as enantiomeric forms, diastereomeric forms and racemic forms, thereof;
wherein
n is 1, 2 or 3;
each R₁, R₂, R₃, R₄ and R₅ is independently selected from the group consisting of hydrogen, optionally substituted C₁₋₆-alkyl, optionally substituted C₃₋₆-cycloalkyl, optionally substituted C₂₋₆-alkenyl, optionally substituted C₄₋₆-alkadienyl, optionally substituted C₂₋₆- alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆- alkylcarbonyl, formyl, C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, amino, monoand di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆- alkylamino-C₁₋₆-alkyl-aminocarbonyl, C₁₋₆-alkylcarbonylamino, amino-C₁₋₆-alkyl- carbonylamino, mono- and di(C₁₋₆-alkyl)amino-C₁₋₆-alkyl-carbonylamino, cyano, guanidino, carbamido, C₁₋₆-alkanoyloxy, C₁₋₆-alkylsulphonyl, C₁₋₆-alkylsulphinyl, C₁₋₆-alkylsulphonyl- oxy, aminosulfonyl, mono- and di(C₁₋₆-alkyl)aminosulfonyl, nitro, optionally substituted C₁₋₆-alkylthio and halogen, where any nitrogen-bound C₁₋₆-alkyl is optionally substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine;
each R₆ and R₇ is independently selected from the group consisting of hydrogen, optionally substituted C₁₋₆-alkyl, optionally substituted C₂₋₆-alkenyl, optionally substituted C₄₋₆- alkadienyl, optionally substituted C₂₋₆-alkynyl, optionally substituted C₁₋₆-alkoxycarbonyl, optionally substituted C₁₋₆-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di(C₁₋₆-alkyl)aminocarbonyl, amino- C₁₋₆-alkyl-aminocarbonyl and mono- and di(C₁₋₆-alkyl)amino- C₁₋₆-alkyl-aminocarbonyl; or R₆ and R₇ may together form a five- or six-membered nitrogen-containing ring;
or a pharmaceutically acceptable derivative thereof, for use in the treatment of a kidney disease.

It is also an aspect to provide use of a composition comprising a compound of formula (I) or (Ia): 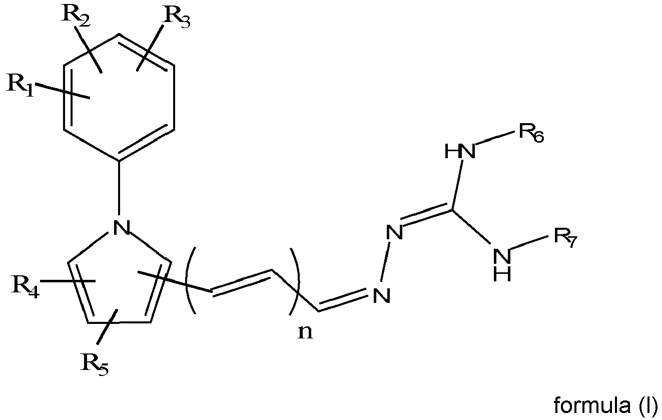 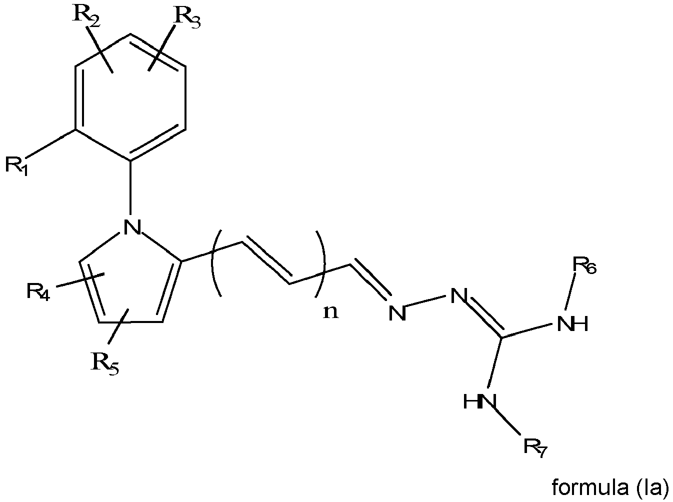 including tautomeric and isomeric forms, such as enantiomeric forms, diastereomeric forms and racemic forms, thereof; wherein each of n, R₁, R₂, R₃, R₄ and R₅, R₆ and R₇ are as defined herein above, or a pharmaceutically acceptable derivative thereof, for the manufacture of a medicament for the treatment of a kidney disease.

It is also an aspect to provide a method for treating a kidney disease, said method comprising one or more steps of administration of a composition comprising a compound of formula (I) or (Ia): 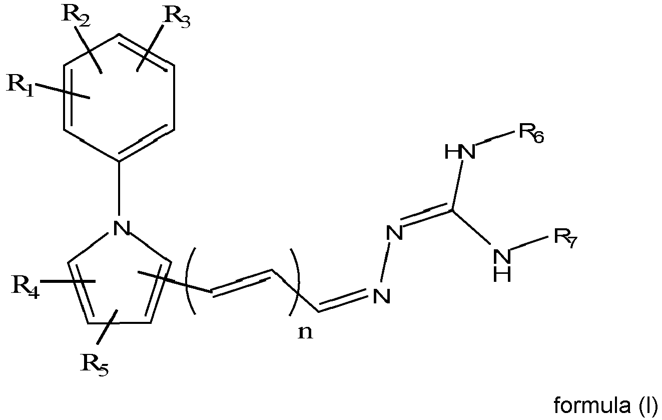 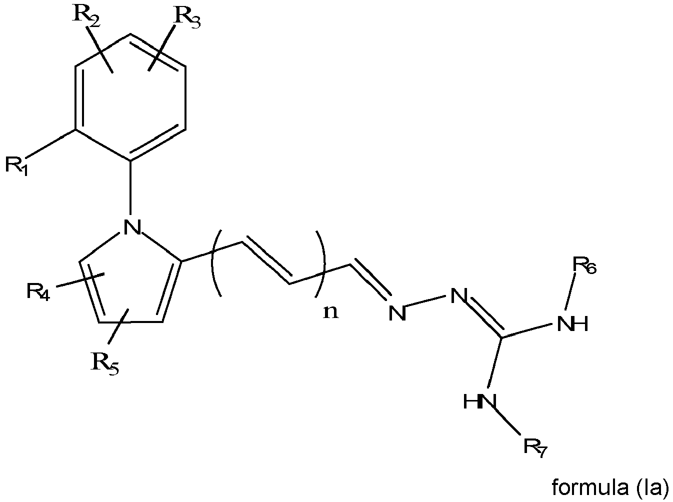 including tautomeric and isomeric forms, such as enantiomeric forms, diastereomeric forms and racemic forms, thereof; wherein each of n, R₁, R₂, R₃, R₄ and R₅, R₆ and R₇ are as defined herein above, or a pharmaceutically acceptable derivative thereof, to an individual in need thereof.

The present disclosure also provides a composition comprising a compound of formula (II): 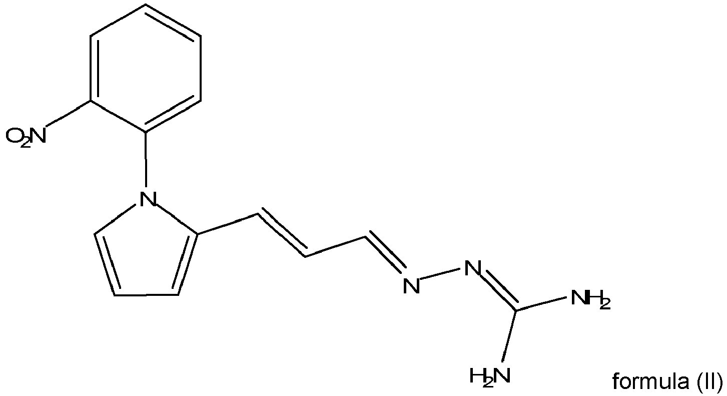 including tautomeric and isomeric forms, such as enantiomeric forms, diastereomeric forms and racemic forms, thereof; or a pharmaceutically acceptable derivative thereof, for use in the treatment of a kidney disease.

In one embodiment there is provided a composition comprising a compound of formula (I), (Ia) or (II) as defined herein for use in the treatment of a kidney disease, wherein said kidney disease present with proteinuria.

Proteinuria is the presence of excess proteins in the urine. In healthy persons, urine contains very little protein; an excess is suggestive of illness. The main mechanisms to cause proteinuria is disease in the kidney (esp. the glomerulus) or kidney damage, increased quantity of proteins in serum (overflow proteinuria), low reabsorption at proximal tubule, certain biological agents and excessive fluid intake.

Proteinuria may be defined as defined as >3.5 g per 1.73 m² body surface area per day, or > 40 mg per square meter body surface area per hour in children.

In one embodiment there is provided a composition comprising a compound of formula (I), (Ia) or (II) as defined herein for use in the treatment of a kidney disease, wherein said kidney disease present with proteinuria defined as >3.5 g per 1.73 m² body surface area per day, or > 40 mg per square meter body surface area per hour in children.

A 'kidney disease presenting with proteinuria' means that proteinuria is an associated symptom or sign of said kidney disease. A 'kidney disease presenting with proteinuria' may also be described as a kidney disease associated with proteinuria or a kidney disease with proteinuria or a proteinuric kidney disease.

In one embodiment there is provided a composition comprising a compound of formula (I), (Ia) or (II) as defined herein for use in the treatment of a kidney disease, wherein said kidney disease is a glomerular disease, such as a disease affecting the podocytes of the glomeruli.

A glomerular disease may also reflect any type of glomerular injury caused by glomerular disease or any other stimuli, such as other underlying diseases or influences.

The term 'caused by' a disease in the present context means that the injury arises or emerges as a cause or result of a disease, such as an underlying disease, i.e. as a result of the pathologic condition.

In one embodiment there is provided a composition comprising a compound of formula (I), (Ia) or (II) as defined herein for use in the treatment of a kidney disease, wherein said kidney disease is nephrotic syndrome (glomerulonephrosis).

Nephrotic syndrome is a collection of symptoms due to kidney damage, including proteinuria, low blood albumin levels, high blood lipids, and significant swelling. Other symptoms may include weight gain, feeling tired, and foamy urine. Complications may include blood clots, infections, and high blood pressure. Causes include a number of kidney diseases and may also occur as a complication of for example diabetes or lupus. The underlying mechanism typically involves damage to the glomeruli of the kidney.

In one embodiment there is provided a composition comprising a compound of formula (I), (Ia) or (II) as defined herein for use in the treatment of a kidney disease, wherein said kidney disease is primary nephrotic syndrome (primary glomerulonephrosis).

In one embodiment said primary nephrotic syndrome is membranous glomerulonephritis (MGN) (or membranous nephropathy (MN)).

In one embodiment said primary nephrotic syndrome is focal segmental glomerulosclerosis (FSGS)

In one embodiment said primary nephrotic syndrome is membranoproliferative glomerulonephritis (MPGN) (mesangiocapillary glomerulonephritis).

In one embodiment said membranoproliferative glomerulonephritis (MPGN) is selected from Type 1 MPGN and Type 2 MPGN.

In one embodiment said primary nephrotic syndrome is rapidly progressive glomerulonephritis (RPGN) (crescentic GN).

In one embodiment said primary nephrotic syndrome is minimal change disease (MCD).

In one embodiment there is provided a composition comprising a compound of formula (I), (Ia) or (II) as defined herein for use in the treatment of a kidney disease, wherein said kidney disease is secondary nephrotic syndrome (secondary glomerulonephrosis).

In one embodiment said secondary nephrotic syndrome is caused by an underlying disease (or simply caused by a disease). The term 'caused by' a disease in the present context means that the nephrotic syndrome arises or emerges as a cause or result of a disease, such as an underlying disease, i.e. as a result of the pathologic condition.

In one embodiment said secondary nephrotic syndrome is caused by an underlying autoimmune disease.

In one embodiment said secondary nephrotic syndrome is caused by an underlying cancer disease.

In one embodiment said secondary nephrotic syndrome is caused by an underlying genetic disorder.

In one embodiment said secondary nephrotic syndrome is caused by an underlying disease selected from the group consisting of: Systemic lupus erythematosus (SLE), Diabetic nephropathy, Sarcoidosis, Sjögren's syndrome, Amyloidosis, Multiple myeloma, Vasculitis, Cancer and Genetic disorders (such as congenital nephrotic syndrome).

Lupus nephritis is an inflammation of the kidneys caused by SLE. Hence, in one embodiment the secondary nephrotic syndrome is caused by lupus nephritis.

In one embodiment said secondary nephrotic syndrome is caused by Diabetic nephropathy.

In one embodiment said secondary nephrotic syndrome is caused by an infection.

In one embodiment said secondary nephrotic syndrome is caused by a urinary tract infection.

In one embodiment said secondary nephrotic syndrome is caused by an infection selected from the group consisting of HIV, syphilis, hepatitis such as hepatitis A, B and C, post-streptococcal infection, urinary schistosomiasis and Ebola.

In one embodiment said secondary nephrotic syndrome is drug-induced; i.e. a drug-induced nephrotic syndrome.

In one embodiment said drug-induced nephrotic syndrome is caused by a drug selected from the group consisting of antibiotics, nonsteroidal anti-inflammatory drugs (NSAID), radiocontrast media, anticancer drugs, antirheumatic drugs, antibody-based therapies, anti-TNF-a therapies, penicillamine, nicotine, lithium carbonate, gold and other heavy metals, ACE inhibitors and opiates (such as heroin).

The term 'caused by' a drug in the present context means that the nephrotic syndrome arises or emerges as a cause or result of the use or administration of a drug.

In one embodiment there is provided a composition comprising a compound of formula (I), (Ia) or (II) as defined herein for use in the treatment of a kidney disease, wherein said kidney disease is an inflammatory kidney disease.

In one embodiment there is provided a composition comprising a compound of formula (I), (Ia) or (II) as defined herein for use in the treatment of a kidney disease, wherein said kidney disease is glomerulonephritis (GN).

In one embodiment said glomerulonephritis is selected from the group consisting of IgA nephropathy (Berger's disease), IgM nephropathy, Post-infectious glomerulonephritis and Thin basement membrane disease.

In one embodiment there is provided a composition comprising a compound of formula (I) or (Ia) as defined herein for use in the treatment of a kidney disease, wherein n = 1.

In one embodiment there is provided a composition comprising a compound of formula (I) or (Ia) as defined herein for use in the treatment of a kidney disease, wherein each R₁, R₂ and R₃ is independently selected from the group consisting of -H, -CH₃, -CF₃, - CCl₃, -O-C₁₋₆ alkyl, -OH, -OCH₃, -NH-C₁₋₆ alkyl, -N(C₁₋₆ alkyl)₂, -S-C₁₋₆ alkyl, -^{t}Bu, -CN, - SO₃-C₁₋₆ alkyl, -NH₂, -NO₂, -CO₂H, -CO₂-C₁₋₆ alkyl, -C(=O)-C₁₋₆ alkyl, -CHO, morpholine, pyrrolidine, pyrrole, or halogen.

In one embodiment there is provided a composition comprising a compound of formula (I) or (Ia) as defined herein for use in the treatment of a kidney disease, wherein R₁ and/or R₂ each is an electron-withdrawing group.

An electron-withdrawing group is understood as an electron-withdrawing element or functional group that draws electron density from neighbouring atoms towards itself, usually by resonance or inductive effects. Whether a functional group is electron-withdrawing or electron-donating can be determined using the Hammett equation, an equation known to the person skilled in the art. Hammett substituent constants, also known as substituent constants σ can be used as a measure of a functional group's ability to draw electron density from neighboring atoms towards itself. An electron-withdrawing group is an electron-withdrawing element or functional group with a substituent constant σ > 0, such as between 0.01 and 0.9 for example 0.78. These values are known in the art and can be found in tables in the scientific literature such as J. Org. Chem., 23, 420 (1958). CF₃, CCl₃, F, Cl, CN and NO₂ are examples of electron-withdrawing groups.

In one embodiment said R₁ is selected from the group consisting of CF₃, CCl₃, F, Cl, CN and NO₂.

In one embodiment said R₂ and/or R₃ each is hydrogen.

In one embodiment each R₄ and R₅ is independently selected from the group consisting of -H, C₁₋₆ alkyl, halogen, -O(C₁₋₆ alkyl), -NH(C₁₋₆ alkyl) and -C(=O)C₁₋₆ alkyl.

In one embodiment each R₆ and R₇ is independently selected from the group consisting of hydrogen, methyl, ethyl and propyl.

In one embodiment said R₆ and R₇ are each hydrogen.

In one embodiment said R₆ is hydrogen and R₇ is methyl or ethyl. In one embodiment said R₆ is hydrogen and R₇ is methyl. In one embodiment said R₆ is hydrogen and R₇ is ethyl. In one embodiment said R₆ and R₇ are both methyl. In one embodiment said R₆ and R₇ are both ethyl.

In one embodiment said R₂, R₃, R₄, R₅, R₆ and R₇ are each hydrogen, and said R₁ is selected from the group consisting of CF₃, CCl₃, F, Cl, NO₂ and CN.

In one embodiment said R₂, R₃, R₄, R₅, R₆ and R₇ are each hydrogen, said R₁ is selected from the group consisting of CF₃, CCl₃, F, Cl, NO₂ or CN, and n = 1.

In one embodiment said R₃ is located on the 4-position.

In one embodiment said R₃ is selected from the group consisting of F, Cl and Br.

In one embodiment said compound is {3-[1-(2-nitrophenyl)-1H-pyrrol-2-yl]-allylidene}-aminoguanidine, preferably (*E*)-*N*-*trans*-{3-[1-(2-nitrophenyl)-1H-pyrrol-2-yl]-allylidene}-aminoguanidine or a pharmaceutically acceptable salt thereof.

In one embodiment there is provided a composition comprising a compound of formula (I), (Ia) or (II) as defined herein for use in the treatment of a kidney disease, wherein the pharmaceutically acceptable derivative thereof is a pharmaceutically acceptable salt of an inorganic acid or an organic acid.

In one embodiment the organic acid is selected from the group consisting of: formic acid, acetic acid, trichloroacetic acid, trifluoroacetic acid, propionic acid, benzoic acid, cinnamic acid, citric acid, fumaric acid, glycolic acid, lactic acid, maleic acid, malic acid, malonic acid, mandelic acid, oxalic acid, picric acid, pyruvic acid, salicylic acid, succinic acid, methanesulfonic acid, ethanesulfonic acid, tartaric acid, ascorbic acid, pamoic acid, bismethylene salicylic acid, ethanedisulfonic acid, gluconic acid, citraconic acid, aspartic acid, stearic acid, palmitic acid, EDTA, glycolic acid, p-aminobenzoic acid, glutamic acid, benzenesulfonic acid and p-toluenesulfonic acid.

In one embodiment said organic acid is acetic acid, succinic acid, tartaric acid or propionic acid.

In one embodiment said inorganic acid is selected from the group consisting of: hydrochloric acid, hydrobromic acid, hydroiodic acid, phosphoric acid, sulphuric acid and nitric acid.

In one embodiment said pharmaceutically acceptable acid is acetic acid.

In one embodiment said compound is {3-[1-(2-nitrophenyl)-1H-pyrrol-2-yl]-allylidene}-aminoguanidinium acetate, preferably (*E*)-*N*-*trans*-{3-[1-(2-nitrophenyl)-1H-pyrrol-2-yl]-allylidene}-aminoguanidinium acetate.

In one embodiment there is provided a composition comprising a compound of formula (la), wherein said R₂, R₃, R₄ and R₅ are each hydrogen, R₆ and R₇ are each independently selected from the group consisting of hydrogen, methyl, ethyl and propyl, said R₁ is selected from the group consisting of CF₃, CCl₃, F, Cl, NO₂ or CN, and n = 1 for use in the treatment of a kidney disease.

In one embodiment there is provided a composition comprising a compound of formula (la), wherein said R₂, R₃, R₄, R₅, R₆ and R₇ are each hydrogen, said R₁ is selected from the group consisting of CF₃, CCl₃, F, Cl, NO₂ or CN, and n = 1 for use in the treatment of a kidney disease.

In one embodiment there is provided a composition comprising a compound of formula (la), wherein said R₂, R₃, R₄, R₅, R₆ and R₇ are each hydrogen, said R₁ is selected from the group consisting of CF₃, CCl₃, F, Cl, NO₂ or CN, and n = 1 for use in the treatment of a kidney disease, wherein said kidney disease present with proteinuria.

In one embodiment there is provided a composition comprising a compound of formula (la), wherein said R₂, R₃, R₄, R₅, R₆ and R₇ are each hydrogen, said R₁ is selected from the group consisting of CF₃, CCl₃, F, Cl, NO₂ or CN, and n = 1 for use in the treatment of nephrotic syndrome. In one embodiment said nephrotic syndrome is selected from the group consisting of primary nephrotic syndrome and secondary nephrotic syndrome.

In one embodiment there is provided a composition comprising a compound of formula (la), wherein said R₂, R₃, R₄, R₅, R₆ and R₇ are each hydrogen, said R₁ is selected from the group consisting of CF₃, CCl₃, F, Cl, NO₂ or CN, and n = 1 for use in the treatment of primary nephrotic syndrome (primary glomerulonephrosis), wherein said primary nephrotic syndrome is selected from the group consisting of: membranous glomerulonephritis (MGN) (or membranous nephropathy (MN)), focal segmental glomerulosclerosis (FSGS), membranoproliferative glomerulonephritis (MPGN) (mesangiocapillary glomerulonephritis) selected from the group consisting of Type 1 MPGN and Type 2 MPGN, rapidly progressive glomerulonephritis (RPGN) (crescentic GN) and minimal change disease (MCD) .

In one embodiment there is provided a composition comprising a compound of formula (la), wherein said R₂, R₃, R₄, R₅, R₆ and R₇ are each hydrogen, said R₁ is selected from the group consisting of CF₃, CCl₃, F, Cl, NO₂ or CN, and n = 1 for use in the treatment of secondary nephrotic syndrome (secondary glomerulonephrosis), wherein said secondary nephrotic syndrome is caused by a disease selected from the group consisting of: an underlying autoimmune disease, Systemic lupus erythematosus (lupus nephritis), Diabetic nephropathy, Sarcoidosis, Sjögren's syndrome, Amyloidosis, Multiple myeloma, Vasculitis, Cancer, Genetic disorders (such as congenital nephrotic syndrome), an infection and Diabetic nephropathy.

In one embodiment there is provided a composition comprising a compound of formula (la), wherein said R₂, R₃, R₄, R₅, R₆ and R₇ are each hydrogen, said R₁ is selected from the group consisting of CF₃, CCl₃, F, Cl, NO₂ or CN, and n = 1 for use in the treatment of drug-induced nephrotic syndrome. In one embodiment said drug-induced nephrotic syndrome is caused by a drug selected from the group consisting of: antibiotics, nonsteroidal anti-inflammatory drugs (NSAID), radiocontrast media, anticancer drugs, antirheumatic drugs, antibody-based therapies, anti-TNF-a therapies, penicillamine, nicotine, lithium carbonate, gold and other heavy metals, ACE inhibitors and opiates (especially heroin).

In one embodiment there is provided a composition comprising a compound of formula (Ia), wherein said R₂, R₃, R₄, R₅, R₆ and R₇ are each hydrogen, said R₁ is selected from the group consisting of CF₃, CCl₃, F, Cl, NO₂ or CN, and n = 1 for use in the treatment of a kidney disease, wherein said kidney disease is selected from the group consisting of: a glomerular disease, a disease affecting the podocytes of the glomeruli, an inflammatory kidney disease and glomerulonephritis (GN); such as a glomerulonephritis selected from the group consisting of: IgA nephropathy (Berger's disease), IgM nephropathy, Post-infectious glomerulonephritis and Thin basement membrane disease.

In one embodiment there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)*-*N*-*trans*-{3-[1-(2-nitrophenyl)-1H-pyrrol-2-yl]-allylidene}-aminoguanidine; and *(E)-N-trans*-{3-[1-(2-nitrophenyl)-1H-pyrrol-2-yl]-allylidene}-aminoguanidinium acetate, or a pharmaceutically acceptable derivative thereof, for use in the treatment of a kidney disease. In one embodiment said kidney disease present with proteinuria.

In one embodiment there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)*-*N*-*trans*-{3-[1-(2-nitrophenyl)-1H-pyrrol-2-yl]-allylidene}-aminoguanidine; and *(E)-N-trans*-{3-[1-(2-nitrophenyl)-1H-pyrrol-2-yl]-allylidene}-aminoguanidinium acetate, or a pharmaceutically acceptable derivative thereof,for use in the treatment of nephrotic syndrome. In one embodiment said nephrotic syndrome is selected from the group consisting of primary nephrotic syndrome and secondary nephrotic syndrome.

In one embodiment there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)*-*N*-*trans*-{3-[1-(2-nitrophenyl)-1H-pyrrol-2-yl]-allylidene}-aminoguanidine; and *(E)-N-trans*-{3-[1-(2-nitrophenyl)-1H-pyrrol-2-yl]-allylidene}-aminoguanidinium acetate, or a pharmaceutically acceptable derivative thereof, for use in the treatment of primary nephrotic syndrome (primary glomerulonephrosis), wherein said primary nephrotic syndrome is selected from the group consisting of: membranous glomerulonephritis (MGN) (or membranous nephropathy (MN)), focal segmental glomerulosclerosis (FSGS), membranoproliferative glomerulonephritis (MPGN) (mesangiocapillary glomerulonephritis) selected from the group consisting of Type 1 MPGN and Type 2 MPGN, rapidly progressive glomerulonephritis (RPGN) (crescentic GN) and minimal change disease (MCD) .

In one embodiment there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)*-*N*-*trans*-{3-[1-(2-nitrophenyl)-1H-pyrrol-2-yl]-allylidene}-aminoguanidine; and *(E)-N-trans*-{3-[1-(2-nitrophenyl)-1H-pyrrol-2-yl]-allylidene}-aminoguanidinium acetate, or a pharmaceutically acceptable derivative thereof, for use in the treatment of secondary nephrotic syndrome (secondary glomerulonephrosis), wherein said secondary nephrotic syndrome is caused by a disease selected from the group consisting of: an underlying autoimmune disease, Systemic lupus erythematosus (lupus nephritis), Diabetic nephropathy, Sarcoidosis, Sjögren's syndrome, Amyloidosis, Multiple myeloma, Vasculitis, Cancer, Genetic disorders (such as congenital nephrotic syndrome), an infection and Diabetic nephropathy.

In one embodiment there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)*-*N*-*trans*-{3-[1-(2-nitrophenyl)-1H-pyrrol-2-yl]-allylidene}-aminoguanidine; and *(E)-N-trans*-{3-[1-(2-nitrophenyl)-1H-pyrrol-2-yl]-allylidene}-aminoguanidinium acetate, or a pharmaceutically acceptable derivative thereof, for use in the treatment of secondary nephrotic syndrome caused by Diabetic nephropathy.

In one embodiment there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)*-*N*-*trans*-{3-[1-(2-nitrophenyl)-1H-pyrrol-2-yl]-allylidene}-aminoguanidine; and *(E)-N-trans*-{3-[1-(2-nitrophenyl)-1H-pyrrol-2-yl]-allylidene}-aminoguanidinium acetate, or a pharmaceutically acceptable derivative thereof, for use in the treatment of drug-induced nephrotic syndrome. In one embodiment said drug-induced nephrotic syndrome is caused by a drug selected from the group consisting of: antibiotics, nonsteroidal anti-inflammatory drugs (NSAID), radiocontrast media, anticancer drugs, antirheumatic drugs, antibody-based therapies, anti-TNF-a therapies, penicillamine, nicotine, lithium carbonate, gold and other heavy metals, ACE inhibitors and opiates (especially heroin).

In one embodiment there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)*-*N*-*trans*-{3-[1-(2-nitrophenyl)-1H-pyrrol-2-yl]-allylidene}-aminoguanidine; and *(E)-N-trans*-{3-[1-(2-nitrophenyl)-1H-pyrrol-2-yl]-allylidene}-aminoguanidinium acetate, or a pharmaceutically acceptable derivative thereof, for use in the treatment of a kidney disease selected from the group consisting of: a glomerular disease, a disease affecting the podocytes of the glomeruli, an inflammatory kidney disease and glomerulonephritis (GN); such as a glomerulonephritis selected from the group consisting of: IgA nephropathy (Berger's disease), IgM nephropathy, Post-infectious glomerulonephritis and Thin basement membrane disease.

Without wishing to be bound to any theory, the compounds of the present disclosure in one embodiment exert their beneficial effects via an effect as an agonist of one or more MC receptors, such as via the MC1R and MC3R, such as via MC1R. The compounds of the present disclosure in one embodiment exert their beneficial effects via an effect as a biased agonist of the MC1R and MC3R.

In one embodiment of the present disclosure there is provided a composition comprising a compound of formula (I), (Ia) or (II) as defined herein, including tautomeric and isomeric forms, such as enantiomeric forms, diastereomeric forms and racemic forms, thereof, for use in the treatment of a kidney disease, wherein said compound is:
i) an agonist of one or more MC receptors,
ii) an agonist of the MC1R and MC3R,
iii) an agonist of MC1R,
iv) an agonist of the MC1R and/or MC3R, but not an agonist of MC2R and MC4R,
v) a biased agonist of the MC1R and MC3R,
vi) a biased agonist of the MC1R and MC3R, that mainly induces ERK1/2 phosphorylation, and insignificantly induces cAMP generation,
vii) a biased agonist of the MC1R and MC3R, that does not stimulate melanogenesis via the MC1R, and/or
viii) an agonist of the MC1R and/or MC3R, with no effect on energy homeostasis such as food intake

In one embodiment the compound of the present disclosure does not reduce food intake, such as does not reduce food intake via the MC3R and/or the MC4R.

In one embodiment the compound of the present disclosure does not induce melanogenesis.

### Combination therapies

The compounds or compositions of the present disclosure may be combined with or comprise one or more additional active ingredients which are understood as other therapeutically effective compounds or pharmaceutically acceptable derivatives thereof.

In one embodiment the composition comprising a compound of formula (I), (Ia) or (II), or a pharmaceutically acceptable derivative thereof, comprises, separately or together, one or more additional active pharmaceutical ingredients.

In one embodiment the composition comprising a compound of formula (I), (Ia) or (II), or a pharmaceutically acceptable derivative thereof, comprises, separately or together, one or more additional active pharmaceutical ingredients used for the treatment of kidney disease.

In one embodiment said one or more additional active pharmaceutical ingredients comprise ingredients used for the treatment of kidney disease.

In one embodiment said one or more additional active pharmaceutical ingredients comprise ingredients used for the treatment of the underlying cause of said kidney disease.

In one embodiment said one or more additional active pharmaceutical ingredients comprise ingredients used for the treatment of the underlying cause of said kidney disease, such as ingredients for treatment of high blood pressure, of high blood cholesterol, and of infection; a low salt diet and limiting fluids.

In one embodiment said one or more additional active pharmaceutical ingredients used for the treatment of kidney disease is selected from the group consisting of angiotensin converting enzyme (ACE) inhibitors, aldosterone antagonist and angiotensin receptor blocker (ARB).

In one embodiment said one or more additional active pharmaceutical ingredients used for the treatment of kidney disease and proteinuria secondary to autoimmune disease is treated with steroids or a steroid-sparing agent plus the use of ACE inhibitors.

In another embodiment said one or more additional active pharmaceutical ingredients comprise ingredients used for the treatment of the underlying cause of the kidney disease.

In one embodiment the combination of additional medicaments has a dose-sparing effect of lowering the required dosage of the medication used in combination with the compound of the present disclosure.

In one embodiment the composition comprising a compound of formula (I), (Ia) or (II) as defined herein, and the additional active ingredient, are administered simultaneously, sequentially or separately.

In one embodiment the composition comprising a compound of formula (I), (Ia) or (II) as defined herein is administered before the additional active ingredient. In another embodiment, the composition comprising a compound of formula (I) as defined herein is administered simultaneously with the additional active ingredient. In yet another embodiment, the composition comprising a compound of formula (I) as defined herein is administered after the additional active ingredient.

### Routes of administration

It will be appreciated that the preferred route of administration will depend on the general condition and age of the subject to be treated, the nature of the condition to be treated, the location of the tissue to be treated in the body and the active ingredient chosen.

In one embodiment the composition comprising a compound of formula (I), (Ia) or (II) as defined herein is administered by systemic administration, local administration, enteral administration or parenteral administration. Appropriate dosage forms for such administration may be prepared by conventional techniques.

### Systemic administration

Systemic administration is capable of introducing the compound into the blood stream to ultimately target the sites of desired action.

Such routes of administration are any suitable routes, such as an enteral route, the oral, rectal, nasal, pulmonary, buccal, sublingual, transdermal, intracisternal, intraperitoneal, and parenteral (including subcutaneous, intramuscular, intrathecal, intravenous and intradermal) route.

In one embodiment, the composition comprising a compound of formula (I), (Ia) or (II) as defined herein is administered by systemic administration.

### Oral administration

Oral administration is normally for enteral drug delivery, wherein the compound is delivered through the enteral mucosa. Syrups and solid oral dosage forms, such as tablets, capsules and the like, are commonly used.

In one embodiment, the composition comprising a compound of formula (I), (Ia) or (II) as defined herein is administered by oral administration.

### Parenteral administration

Parenteral administration is any administration route not being the oral/enteral route whereby the medicament avoids first-pass degradation in the liver. Accordingly, parenteral administration includes any injections and infusions, for example bolus injection or continuous infusion, such as intravenous administration, intramuscular administration, subcutaneous administration. Furthermore, parenteral administration includes inhalations and topical administration.

Accordingly, the compound may be administered topically to cross any mucosal membrane of an animal to which the biologically active substance is to be given, e.g. in the nose, vagina, eye, mouth, genital tract, lungs, gastrointestinal tract, or rectum, preferably the mucosa of the nose, or mouth, and accordingly, parenteral administration may also include buccal, sublingual, nasal, rectal, vaginal and intraperitoneal administration as well as pulmonal and bronchial administration by inhalation or installation. Also, the compound may be administered topically to cross the skin.

The subcutaneous and intramuscular forms of parenteral administration are generally preferred.

### Local treatment

The compound as disclosed herein is in one embodiment used as a local treatment, i.e. is introduced directly to the site(s) of action. Accordingly, the compound may be applied to the skin or mucosa directly, or the compound may be injected into the site of action, for example into the diseased tissue or to an end artery leading directly to the diseased tissue.

### Dosage

According to the present disclosure, the composition comprising a compound of formula (I), (Ia) or (II) is administered to individuals in need of treatment in pharmaceutically effective doses. A therapeutically effective amount of a compound is an amount sufficient to cure, prevent, reduce the risk of, alleviate or partially arrest the clinical manifestations of a given disease and its complications. The amount that is effective for a particular therapeutic purpose will depend on the severity and the sort of the disorder as well as on the weight and general state of the subject. The compound may be administered one or several times per day, such as from 1 to 8 times per day, such as from 1 to 6 times per day, such as from 1 to 5 times per day, such as from 1 to 4 times per day, such as from 1 to 3 times per day, such as from 1 to 2 times per day, such as from 2 to 4 times per day, such as from 2 to 3 times per day. Alternatively, the compound may be administered less than once a day, for example once a day, such as once every second day, for example once every third day, such as once every fourth day, for example once every fifth day, such as once every sixth day, for example once every week.

In one embodiment the composition comprising a compound of formula (I) as defined herein is administered in a therapeutically effective amount, such as in an amount of 1 mg to 1000 mg compound of formula (I), (Ia) or (II) per day.

It follows that in one embodiment the compound is administered in an amount of 1 mg to 1000 mg, such as 1 to 5 mg, 5 to 10 mg, 10 to 15 mg, 15 to 20 mg, 20 mg, 20 to 30 mg, 30 to 60 mg, 60 to 80 mg, 80 to 100 mg, 100 to 130 mg, 130 to 160 mg, 160 to 200 mg, 200 to 240 mg, 240 to 280 mg, 280 to 320 mg, 320 to 360 mg, 360 to 400 mg, 400 to 440 mg, 440 to 500 mg, 500 to 560 mg, 560 to 620 mg, 620 to 680 mg, 680 to 740 mg, 740 to 800 mg, 800 to 860 mg, 860 to 920 mg, 920 to 980 mg, 980 to 1000 mg, for example 500 to 1000 mg per day.

Per day means the dosage may be given in one dosage or divided in multiple dosages per day, including once a day (QD), twice a day (BID) and/or three times a day (TID).

In another embodiment the compound is administered in an amount of 0.01 mg/kg bodyweight to 40 mg/ kg bodyweight, such as 0.01 mg/ kg bodyweight to 0.05 mg/ kg bodyweight, 0.05 to 0.1 mg/ kg bodyweight, 0.1 to 0.5 mg/ kg bodyweight, 0.5 mg to 1 mg/ kg bodyweight, 1 to 2 mg/ kg bodyweight, 2 to 3 mg/ kg bodyweight, 3 to 5 mg/ kg bodyweight, 5 to 10 mg/ kg bodyweight, 10 to 15 mg/ kg bodyweight, 15 to 20 mg/ kg bodyweight, 20 to 30 mg/ kg bodyweight, for example 30 to 40 mg/ kg bodyweight.

### Formulation

In one embodiment the composition comprising a compound of formula (I), (Ia) or (II) as defined herein is a pharmaceutical composition, such as a pharmaceutically safe composition. The composition comprising a compound of formula (I), (Ia) or (II)) as defined herein may be administered in any suitable way e.g. orally, sublingually, or parenterally, and it may be presented in any suitable form for such administration, e.g. in the form of solutions, suspension, aerosols, tablets, capsules, powders, syrups, implant or dispersions for injection.

In one embodiment, the composition comprising a compound of formula (I), (Ia) or (II) as defined herein is formulated as a suspension.

In one embodiment, the composition comprising a compound of formula (I), (Ia) or (II) as defined herein is formulated as an oral dose form, such as a solid oral dose form or pharmaceutical entity, such as tablets or capsules, or a liquid oral dose form. Methods for the preparation of solid pharmaceutical preparations are well known in the art.

In another embodiment the composition comprising a compound of formula (I), (Ia) or (II) as defined herein is formulated as an injectable dose form.

In one embodiment the composition comprising a compound of formula (I), (Ia) or (II) as defined herein is formulated in the form of a solid pharmaceutical entity, suitably as a tablet or a capsule

The compound (I), (Ia) or (II) as the free base or the salt thereof may be administered alone or in combination with pharmaceutically acceptable carriers or excipients, in either single or multiple doses. The pharmaceutical compositions may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, Pa., 1995.

### References

Lindskog et al. 2010 "Melanocortin 1 Receptor Agonists Reduce Proteinuria". J Am Soc Nephrol 21: 1290-1298, 2010.

Qiao et al. 2016 "MC1R is dispensable for the proteinuria reducing and glomerular protective effect of melanocortin therapy". Nature Scientific Reports 6:27589

Montero-Melendez et al. 2015 "Biased Agonism as a Novel Strategy To Harness the Proresolving Properties of Melanocortin Receptors without Eliciting Melanogenic Effects". J Immunol 2015; 194:3381-3388.

### Items

1. A composition comprising a compound of formula (I) or (Ia): 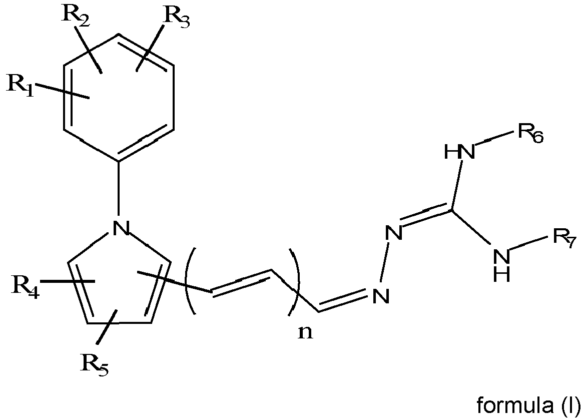 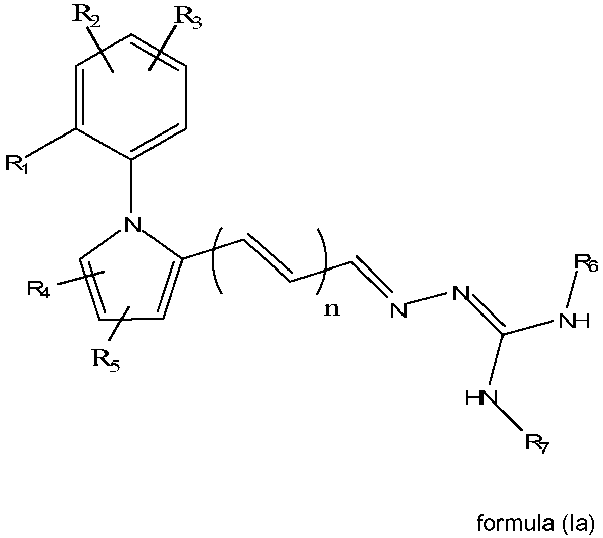
   including tautomeric and isomeric forms, such as enantiomeric forms, diastereomeric forms and racemic forms, thereof;
   wherein
   n is 1, 2 or 3;
   each R₁, R₂, R₃, R₄ and R₅ is independently selected from the group consisting of hydrogen, optionally substituted C₁₋₆-alkyl, optionally substituted C₃₋₆-cycloalkyl, optionally substituted C₂₋₆-alkenyl, optionally substituted C₄₋₆-alkadienyl, optionally substituted C₂₋₆- alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆- alkylcarbonyl, formyl, C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally
   substituted heterocyclyloxy, optionally substituted heterocyclylcarbonyl, optionally substituted heterocyclylamino, heterocyclylsulphonylamino, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkylamino-C₁₋₆-alkyl-aminocarbonyl, C₁₋₆-alkylcarbonylamino, amino-C₁₋₆-alkylcarbonylamino, mono- and di(C₁₋₆-alkyl)amino-C₁₋₆-alkyl-carbonylamino, cyano, guanidino, carbamido, C₁₋₆-alkanoyloxy, C₁₋₆-alkylsulphonyl, C₁₋₆-alkylsulphinyl, C₁₋₆-alkylsulphonyl- oxy, aminosulfonyl, mono- and di(C₁₋₆-alkyl)aminosulfonyl, nitro, optionally substituted C₁₋₆-alkylthio and halogen, where any nitrogen-bound C₁₋₆-alkyl is optionally substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine;
   each R₆ and R₇ is independently selected from the group consisting of hydrogen, optionally substituted C₁₋₆-alkyl, optionally substituted C₂₋₆-alkenyl, optionally substituted C₄₋₆- alkadienyl, optionally substituted C₂₋₆-alkynyl, optionally substituted C₁₋₆-alkoxycarbonyl, optionally substituted C₁₋₆-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di(C₁₋₆-alkyl)aminocarbonyl, amino- C₁₋₆-alkylaminocarbonyl and mono- and di(C₁₋₆-alkyl)amino- C₁₋₆-alkyl-aminocarbonyl; or R₆ and R₇ may together form a five- or six-membered nitrogen-containing ring;
   or a pharmaceutically acceptable derivative thereof,
   for use in the treatment of a kidney disease.
2. The composition for use according to item 1, wherein said kidney disease presents with proteinuria.
3. The composition for use according to any of the preceding items, wherein said kidney disease is a glomerular disease.
4. The composition for use according to any of the preceding items, wherein said kidney disease is a disease affecting the podocytes of the glomeruli.
5. The composition for use according to any of the preceding items, wherein said kidney disease is nephrotic syndrome (glomerulonephrosis).
6. The composition for use according to any of the preceding items, wherein said nephrotic syndrome is primary nephrotic syndrome (primary glomerulonephrosis).
7. The composition for use according to item 6, wherein said primary nephrotic syndrome is membranous glomerulonephritis (MGN) (or membranous nephropathy (MN)).
8. The composition for use according to item 6, wherein said primary nephrotic syndrome is focal segmental glomerulosclerosis (FSGS).
9. The composition for use according to item 6, wherein said primary nephrotic syndrome is membranoproliferative glomerulonephritis (MPGN) (mesangiocapillary glomerulonephritis).
10. The composition for use according to item 9, wherein said membranoproliferative glomerulonephritis (MPGN) is selected from Type 1 MPGN and Type 2 MPGN.
11. The composition for use according to item 6, wherein said primary nephrotic syndrome is rapidly progressive glomerulonephritis (RPGN) (crescentic GN).
12. The composition for use according to item 6, wherein said primary nephrotic syndrome is minimal change disease (MCD).
13. The composition for use according to item 5, wherein said nephrotic syndrome is secondary nephrotic syndrome (secondary glomerulonephrosis).
14. The composition for use according to item 13, wherein said secondary nephrotic syndrome is caused by an underlying autoimmune disease.
15. The composition for use according to item 13, wherein said secondary nephrotic syndrome is caused by an underlying disease selected from the group consisting of: Systemic lupus erythematosus (lupus nephritis), Diabetic nephropathy, Sarcoidosis, Sjögren's syndrome, Amyloidosis, Multiple myeloma, Vasculitis, Cancer and Genetic disorders (such as congenital nephrotic syndrome).
16. The composition for use according to item 13, wherein said secondary nephrotic syndrome is caused by Diabetic nephropathy.
17. The composition for use according to item 13, wherein said secondary nephrotic syndrome is caused by an infection.
18. The composition for use according to item 13, wherein said secondary nephrotic syndrome is caused by a urinary tract infection.
19. The composition for use according to item 17, wherein said infection is selected from the group consisting of HIV, syphilis, hepatitis such as hepatitis A, B and C, post-streptococcal infection, urinary schistosomiasis and Ebola.
20. The composition for use according to item 13, wherein said secondary nephrotic syndrome is drug-induced, i.e. is drug-induced nephrotic syndrome.
21. The composition for use according to item 20, wherein said drug-induced nephrotic syndrome is caused by a drug selected from the group consisting of antibiotics, nonsteroidal anti-inflammatory drugs (NSAID), radiocontrast media, anticancer drugs, antirheumatic drugs, antibody-based therapies, anti-TNF-a therapies, penicillamine, nicotine, lithium carbonate, gold and other heavy metals, ACE inhibitors and opiates (especially heroin).
22. The composition for use according to any of the preceding items, wherein said kidney disease is an inflammatory kidney disease.
23. The composition for use according to any of the preceding items, wherein said kidney disease is glomerulonephritis (GN).
24. The composition for use according to item 23, wherein said glomerulonephritis is selected from the group consisting of IgA nephropathy (Berger's disease), IgM nephropathy, Post-infectious glomerulonephritis and Thin basement membrane disease.
25. The composition for use according to any of the preceding items, wherein n = 1.
26. The composition for use according to any of the preceding items, wherein each R₁, R₂ and R₃ is independently selected from the group consisting of -H, -CH₃, - CF₃, -CCl₃, -O-C₁₋₆ alkyl, -OH, -OCH₃, -NH-C₁₋₆ alkyl, -N(C₁₋₆ alkyl)₂, -S-C₁₋₆ alkyl, -^{t}Bu, -CN, -SO₃-C₁₋₆ alkyl, -NH₂, -NO₂, -CO₂H, -CO₂-C₁₋₆ alkyl, -C(=O)-C₁₋₆ alkyl, -CHO, morpholine, pyrrolidine, pyrrole, or halogen.
27. The composition for use according to any of the preceding items, wherein R₁ and/or R₂ is an electron-withdrawing group.
28. The composition for use according to any of the preceding items, wherein R₁ is CF₃, CCl₃, F, Cl, CN or NO₂.
29. The composition for use according to any of the preceding items, wherein R₂ and/or R₃ is hydrogen.
30. The composition for use according to any of the preceding items, wherein each R₄ and R₅ is independently selected from the group consisting of -H, C₁₋₆ alkyl, halogen, -O(C₁₋₆ alkyl), -NH(C₁₋₆ alkyl) and -C(=O)C₁₋₆ alkyl.
31. The composition for use according to any of the preceding items, wherein each R₆ and R₇ is independently selected from hydrogen, methyl, ethyl or propyl.
32. The composition for use according to any of the preceding items, wherein R₆ and R₇ are hydrogen.
33. The composition for use according to any of the preceding items, wherein R₂, R₃, R₄, R₅, R₆ and R₇ are hydrogen, and wherein R₁ is CF₃, CCl₃, F, Cl, NO₂ or CN.
34. The composition for use according to any of the preceding items, wherein R₂, R₃, R₄, R₅, R₆ and R₇ are hydrogen, and wherein R₁ is CF₃, CCl₃, F, Cl, NO₂ or CN, and wherein n = 1.
35. The composition for use according to any of the preceding items, wherein R₃ is located on the 4-position.
36. The composition for use according to any of the preceding items, wherein R₃ is selected from F, Cl or Br.
37. The composition for use according to any of the preceding items, wherein the compound is of formula (II) 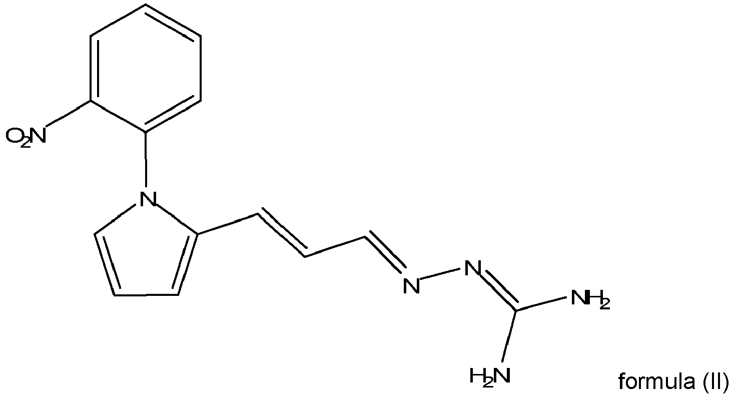 including tautomeric and isomeric forms, such as enantiomeric forms, diastereomeric forms and racemic forms, thereof; or a pharmaceutically acceptable derivative thereof.
38. The composition for use according to any of the preceding items, wherein said compound is selected from the group consisting of {3-[1-(2-nitrophenyl)-1 H-pyrrol-2-yl]-allylidene}-aminoguanidine and (*E*)-*N-trans*-{3-[1-(2-nitrophenyl)-1H-pyrrol-2-yl]-allylidene}-aminoguanidine, or a pharmaceutically acceptable salt thereof.
39. The composition for use according to any of the preceding items, wherein said pharmaceutically acceptable derivative thereof is a pharmaceutically acceptable salt of an inorganic acid or an organic acid.
40. The composition for use according to item 39, wherein said organic acid is selected from the group consisting of: formic acid, acetic acid, trichloroacetic acid, trifluoroacetic acid, propionic acid, benzoic acid, cinnamic acid, citric acid, fumaric acid, glycolic acid, lactic acid, maleic acid, malic acid, malonic acid, mandelic acid, oxalic acid, picric acid, pyruvic acid, salicylic acid, succinic acid, methanesulfonic acid, ethanesulfonic acid, tartaric acid, ascorbic acid, pamoic acid, bismethylene salicylic acid, ethanedisulfonic acid, gluconic acid, citraconic acid, aspartic acid, stearic acid, palmitic acid, EDTA, glycolic acid, p-aminobenzoic acid, glutamic acid, benzenesulfonic acid and p-toluenesulfonic acid.
41. The composition for use according to any items 39-40, wherein said organic acid is acetic acid, succinic acid, tartaric acid or propionic acid.
42. The composition for use according to item 39, wherein said inorganic acid is selected from the group consisting of: hydrochloric acid, hydrobromic acid, hydroiodic acid, phosphoric acid, sulphuric acid and nitric acid.
43. The composition for use according to item 41, wherein said organic acid is acetic acid.
44. The composition for use according to any of the preceding items, wherein said compound is selected from the group consisting of {3-[1-(2-nitrophenyl)-1H-pyrrol-2-yl]-allylidene}- aminoguanidinium acetate and (*E*)-*N-trans*-{3-[1-(2-nitrophenyl)-1H-pyrrol-2-yl]-allylidene}- aminoguanidinium acetate.
45. The composition for use according to any of the preceding items, wherein said compound:
   i) is an agonist of one or more MC receptors,
   ii) is an agonist of the MC1R and MC3R,
   iii) is an agonist of MC1R,
   iv) is an agonist of the MC1R and/or MC3R, but is not an agonist of MC2R and MC4R,
   v) is a biased agonist of the MC1R and MC3R,
   vi) is a biased agonist of the MC1R and MC3R, that mainly induces ERK1/2 phosphorylation, and insignificantly induces cAMP generation,
   vii) is a biased agonist of the MC1R and MC3R, that does not stimulate melanogenesis via the MC1R,
   viii) is an agonist of the MC1R and/or MC3R, with no effect on energy homeostasis such as food intake,
   ix) does not reduce food intake, such as does not reduce food intake via the MC3R and/or the MC4R, and/or
   x) does not induce melanogenesis.
46. The composition for use according to any of the preceding items, wherein said composition comprises, separately or together, one or more additional active pharmaceutical ingredients, such as one or more additional active pharmaceutical ingredients used for the treatment of kidney disease.
47. The composition for use according to any of the preceding items, wherein said compound is administered in an amount of 1 mg to 1000 mg per day, such as 1 to 5 mg, 5 to 10 mg, 10 to 15 mg, 15 to 20 mg, 20 to 30 mg, 30 to 60 mg, 60 to 80 mg, 80 to 100 mg, 100 to 130 mg, 130 to 160 mg, 160 to 200 mg, 200 to 240 mg, 240 to 280 mg, 280 to 320 mg, 320 to 360 mg, 360 to 400 mg, 400 to 440 mg, 440 to 500 mg, 500 to 560 mg, 560 to 620 mg, 620 to 680 mg, 680 to 740 mg, 740 to 800 mg, 800 to 860 mg, 860 to 920 mg, 920 to 980 mg, 980 to 1000 mg, for example 500 to 1000 mg per day.
48. The composition for use according to any of the preceding items, wherein said composition is pharmaceutically safe.

### Examples

### Methods:

Passive Heymann Nephritis was introduced to Male Sprague-Dawley rats by administration of anti-Fx1A serum (PTX-002S, Probetex, San Antonio, TX, USA) Probetex, lot 169-6H) on study day 0 (1 ml serum) and on study day 7 (0.25 mL serum) into the jugular vein by slow injection during isoflurane anaesthesia. This dose regiment has previously (Lindskog et al, J Am Soc Nephrol. 2010; 21:1290-1298) been shown to induce significant proteinuria. Pharmacological intervention with control compound or test compound was initiated on day 14.

In one experiment *(experiment* 1) test compound 1 dissolved in isotonic saline was given once daily by intraperitoneal (ip) injections at a dose of 10 mg/kg (n=12) with animals treated with isotonic saline injections as controls (n=12).

In another experiment *(experiment 2)* test compound 1 was given once daily by oral gavage. Test compound 1 was given as a suspension in 10% hydroxypropyl-betacyclodextrin in water. Two doses of test compound 1 were tested: 20 mg/kg/day (n=8) and 40 mg/kg/day (n=8). In this experiment treatment effect of test compound 1 was compared to the effect of ACTH(1-24) as ACTH from the literature has been described to have treatment effects on proteinuria in the applied experimental model. For the positive control the rats (n=6) received ACTH(1-24) (Bachem cat no H-1150) treatment given at a dose of 10 µg/kg day by continuous subcutaneous infusion by use of Alzet osmotic mini-pumps for 4 weeks. This treatment regimen has previously been shown to induce around 50% reduction in proteinuria compared to untreated controls (Lindskog et al, J Am Soc Nephrol. 2010; 21:1290-1298).

In experiment 1 twenty-four hours urine collection were performed at three occasions, at day 14 following induction of nephritis, i.e. at the time point where pharmacological intervention was initiated, at day 28 and at day 42 following induction of nephritis. For urine collection the rats were placed in metabolic cages followed by a blood sample for measurement of plasma concentrations of creatinine and albumin. Likewise, the urine concentration of creatinine and albumin were determined and so was the volume of the 24-hours urine production.

In experiment 2 twenty-four hours urine collections was conducted at day 42 following induction if nephritis, i.e. following 28 days treatment with test compound 1 or ACTH(1-24). As in experiment 1 the rats were placed in metabolic cages followed by a blood sample for measurement of plasma concentrations of creatinine and albumin. Likewise, the urine concentration of creatinine and albumin were determined and so was the volume of the 24-hours urine production.

The blood sample was taken from the abdominal vein during pentobarbital (0.2-0.3 mL/rat ip; Dolethal, Vetoquinol via Centravet, Lapalisse, France). The collected blood was transferred into tubes coated with EDTA and centrifuged (10 minutes, 1000 g, 4°C) for plasma collection. Plasma samples were stored at -80°C until quantification of creatinine and Albumin.

Albumin and creatinine in urine and plasma were quantified using a ABX Pentra 400 Clinical Chemistry analyzer (HORIBA).

*Albumin excretion rate* was determined as follows:
24 hours urine production x concentration of Albumin in urine.

*Creatinine clearance* as estimate of glomerular filtration rate was determined as follows:
(Urine concentration of creatinine x 24 hours urine production)/plasma concentration of creatinine

*Fractional Albumin excretion* was determined as follows:
((Urine concentration of urine x 24 hours urine production)/plasma concentration of albumin)/Creatinine Clearance x100

All data presented as mean ± Standard error of mean (SE). Statistical analyses were made by standard unpaired t-test, with p<0.05 considered statistical significant

### Results:

Test compound 1 = AP1189 = (*(E)-N-trans*-{3-[1-(2-nitrophenyl)-1H-pyrrol-2-yl]-allylidene}-aminoguanidium acetate.

ACTH(1-24) = Adrenocorticotropic Hormone Fragment 1-24, human; Tetracosactide. Potent ACTH/MC2R agonist (EC50 = 5.5 nM). Stimulates glucocorticoid release from adrenal glands. Sequence: Ser-Tyr-Ser-Met-Glu-His-Phe-Arg-Trp-Gly-Lys-Pro-Val-Gly-Lys-Lys-Arg-Arg-Pro-Val-Lys-Val-Tyr-Pro.

### Experiment 1:

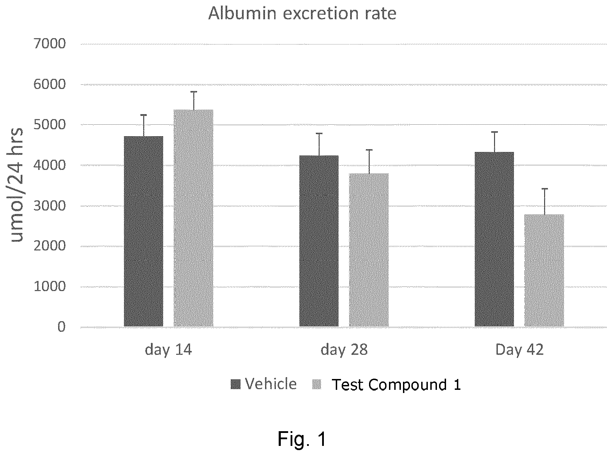

Compared to control rats, rats treated with test compound 1 have a significant reduction in proteinuria at day 28 and day 42. At day 28 rats treated with test compound 1 had 31±8% reduction in albumin excretion rate and at day 42 a statistically significant 48±10% reduction in albumin excretion rate. In the control rats the albumin excretion rate was unchanged throughout (at day 28 96±11% of day 14 and at day 42 102±14% of day 14) (See Fig. 1).

A comparable picture was identified when the fractional album excretion was evaluated: at day 28 FEAlb was reduced by 48±5% and at day 42 by 45±11%. (No figure)

### Experiment 2:

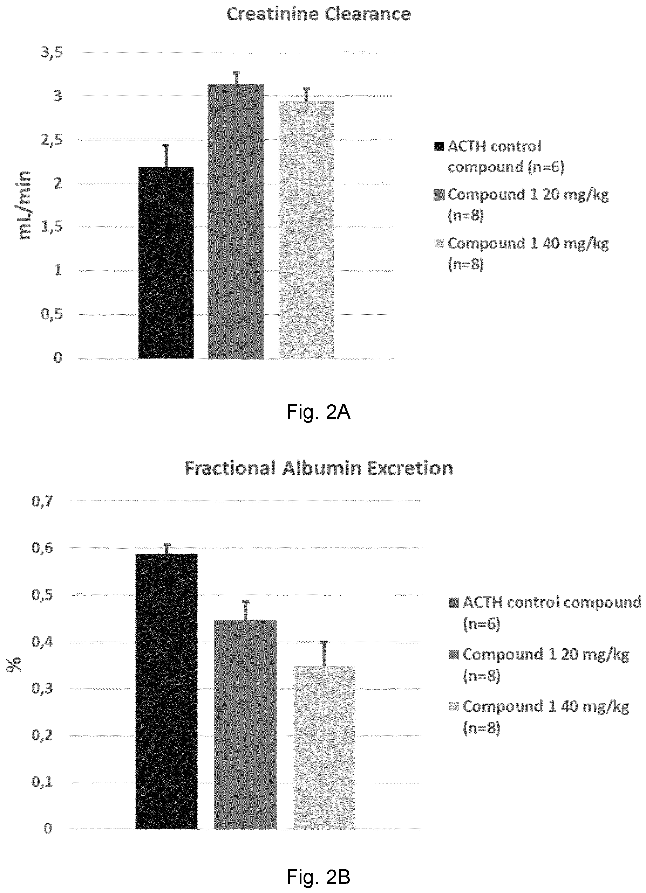

The animals treated with test compound 1 had a significantly higher GFR (creatinine clearance) following 4 weeks treatment than the rats treated with the positive control (ACTH 1-24 at 10 mg/day): GFR in rats treated with positive control: 2.19 ±0.24 mL/min compared to test compound 1 at 20 mg/kg: 3.12±0.14 mL/min (p=0.002 vs positive control) and test compound 1 at 40 mg/kg: 2.94±0.14 mL/24 min (p=0.01 vs positive control) (See Fig. 2A).

Treatment with test compound 1 significantly reduced the fraction albumin excretion (FEAlb) when compared to the positive control (ACTH 1-24 at 10 mg/day): FEAlb in positive control treated rats: 0.59±0.02% vs test compound 1 at 20 mg/kg: 0.045±0.04% (p=0.007 vs positive control) and test compound 1 at 40 mg/kg: 0.35±0.05% (p=0.001 vs positive control) (See Fig. 2B).

## Claims

1. A composition comprising a compound of formula (I) or (Ia): 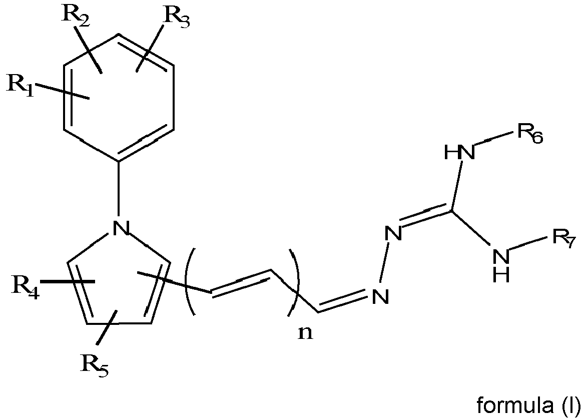 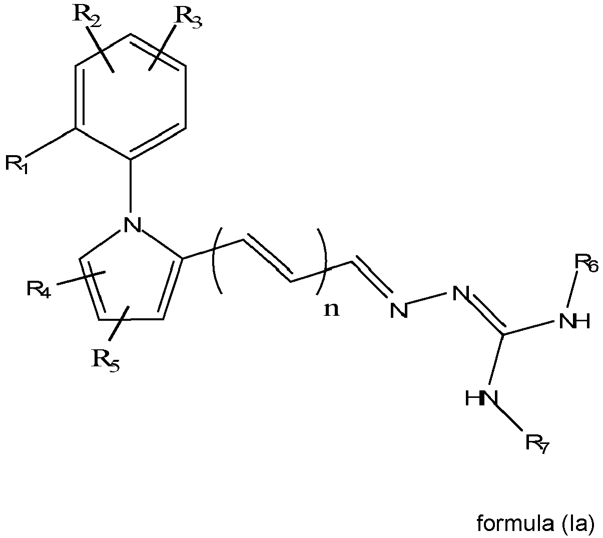
including tautomeric forms, enantiomeric forms, diastereomeric forms, and racemic forms thereof;
wherein
n is 1, 2 or 3;
each R₁, R₂, R₃, R₄ and R₅ is independently selected from the group consisting of hydrogen, optionally substituted C₁₋₆-alkyl, optionally substituted C₃₋₆-cycloalkyl, optionally substituted C₂₋₆-alkenyl, optionally substituted C₄₋₆-alkadienyl, optionally substituted C₂₋₆- alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆- alkylcarbonyl, formyl, C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkylamino-C₁₋₆-alkyl-aminocarbonyl, C₁₋₆-alkylcarbonylamino, amino-C₁₋₆-alkylcarbonylamino, mono- and di(C₁₋₆-alkyl)amino-C₁₋₆-alkyl-carbonylamino, cyano, guanidino, carbamido, C₁₋₆-alkanoyloxy, C₁₋₆-alkylsulphonyl, C₁₋₆-alkylsulphinyl, C₁₋₆-alkylsulphonyl- oxy, aminosulfonyl, mono- and di(C₁₋₆-alkyl)aminosulfonyl, nitro, optionally substituted C₁₋₆-alkylthio and halogen, where any nitrogen-bound C₁₋₆-alkyl is optionally substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine;
each R₆ and R₇ is independently selected from the group consisting of hydrogen, optionally substituted C₁₋₆-alkyl, optionally substituted C₂₋₆-alkenyl, optionally substituted C₄₋₆- alkadienyl, optionally substituted C₂₋₆-alkynyl, optionally substituted C₁₋₆-alkoxycarbonyl, optionally substituted C₁₋₆-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di(C₁₋₆-alkyl)aminocarbonyl, amino- C₁₋₆-alkylaminocarbonyl and mono- and di(C₁₋₆-alkyl)amino- C₁₋₆-alkyl-aminocarbonyl; or R₆ and R₇ may together form a five- or six-membered nitrogen-containing ring;
or a pharmaceutically acceptable derivative thereof,
for use in the treatment of a kidney disease.

2. The composition for use according to claim 1, wherein said kidney disease presents with proteinuria.

3. The composition for use according to any of the preceding claims, wherein said kidney disease is a glomerular disease, and/or a disease affecting the podocytes of the glomeruli.

4. The composition for use according to any of the preceding claims, wherein said kidney disease is nephrotic syndrome (glomerulonephrosis), such as primary nephrotic syndrome (primary glomerulonephrosis) or secondary nephrotic syndrome (secondary glomerulonephrosis).

5. The composition for use according to claim 4, wherein the primary nephrotic syndrome is membranous glomerulonephritis (MGN) (membranous nephropathy (MN)), focal segmental glomerulosclerosis (FSGS), membranoproliferative glomerulonephritis (MPGN) (mesangiocapillary glomerulonephritis) (such as MPGN selected from Type 1 MPGN and Type 2 MPGN), rapidly progressive glomerulonephritis (RPGN) (crescentic GN), or minimal change disease (MCD).

6. The composition for use according to claim 4, wherein said secondary nephrotic syndrome is caused by an underlying autoimmune disease.

7. The composition for use according to claim 4, wherein said secondary nephrotic syndrome is caused by an underlying disease selected from the group consisting of: Systemic lupus erythematosus (lupus nephritis), Diabetic nephropathy, Sarcoidosis, Sjögren's syndrome, Amyloidosis, Multiple myeloma, Vasculitis, Cancer and Genetic disorders (such as congenital nephrotic syndrome).

8. The composition for use according to claim 4, wherein said secondary nephrotic syndrome is caused by an infection such as a urinary tract infection, optionally wherein said infection is selected from the group consisting of HIV, syphilis, hepatitis such as hepatitis A, B and C, post-streptococcal infection, urinary schistosomiasis and Ebola.

9. The composition for use according to claim 4, wherein said secondary nephrotic syndrome is drug-induced, i.e. is drug-induced nephrotic syndrome, optionally wherein said drug-induced nephrotic syndrome is caused by a drug selected from the group consisting of antibiotics, nonsteroidal anti-inflammatory drugs (NSAID), radiocontrast media, anticancer drugs, antirheumatic drugs, antibody-based therapies, anti-TNF-a therapies, penicillamine, nicotine, lithium carbonate, gold and other heavy metals, ACE inhibitors and opiates (especially heroin).

10. The composition for use according to any of the preceding claims, wherein said kidney disease is an inflammatory kidney disease.

11. The composition for use according to any of the preceding claims, wherein said kidney disease is glomerulonephritis (GN), optionally wherein said glomerulonephritis is selected from the group consisting of IgA nephropathy (Berger's disease), IgM nephropathy, Post-infectious glomerulonephritis and Thin basement membrane disease.

12. The composition for use according to any of the preceding claims, wherein n = 1.

13. The composition for use according to any of the preceding claims, wherein the compound is of formula (II) 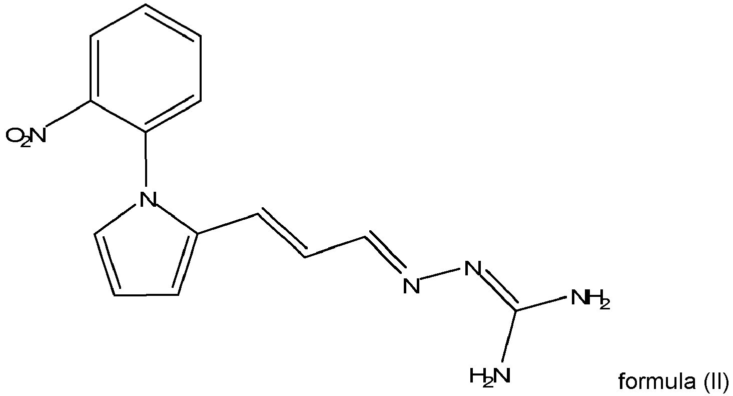 including tautomeric forms, enantiomeric forms, diastereomeric forms, and racemic forms thereof; or a pharmaceutically acceptable derivative thereof.

14. The composition for use according to any one of the preceding claims, wherein said pharmaceutically acceptable derivative thereof is a pharmaceutically acceptable salt of an inorganic acid selected from the group consisting of hydrochloric acid, hydrobromic acid, hydroiodic acid, phosphoric acid, sulphuric acid and nitric acid, or a pharmaceutically acceptable salt of an organic acid selected from the group consisting of formic acid, acetic acid, trichloroacetic acid, trifluoroacetic acid, propionic acid, benzoic acid, cinnamic acid, citric acid, fumaric acid, glycolic acid, lactic acid, maleic acid, malic acid, malonic acid, mandelic acid, oxalic acid, picric acid, pyruvic acid, salicylic acid, succinic acid, methanesulfonic acid, ethanesulfonic acid, tartaric acid, ascorbic acid, pamoic acid, bismethylene salicylic acid, ethanedisulfonic acid, gluconic acid, citraconic acid, aspartic acid, stearic acid, palmitic acid, EDTA, glycolic acid, p-aminobenzoic acid, glutamic acid, benzenesulfonic acid and p-toluenesulfonic acid, such as acetic acid, succinic acid, tartaric acid or propionic acid.

15. The composition for use according to any of the preceding claims, wherein said compound:
i) is an agonist of one or more MC receptors,
ii) is an agonist of the and MC3R,
iii) is an agonist of MC1R,
iv) is an agonist of the MC1R and/or MC3R, but is not an agonist of MC2R and MC4R,
v) is a biased agonist of the MC1R and MC3R,
vi) is a biased agonist of the MC1R and MC3R, that mainly induces ERK1/2 phosphorylation, and insignificantly induces cAMP generation,
vii) is a biased agonist of the MC1R and MC3R, that does not stimulate melanogenesis via the MC1R,
viii) is an agonist of the MC1R and/or MC3R, with no effect on energy homeostasis such as food intake,
ix) does not reduce food intake, such as does not reduce food intake via the MC3R and/or the MC4R, and/or
x) does not induce melanogenesis.
